# FASCICULE DE BREVET EUROPEEN

(11) **EP 0 659 400 B1**
(45) Date de publication et mention de la délivrance du brevet: **19.03.1997**
(21) Numéro de dépôt: 94402571.7
(22) Date de dépôt: 14.11.1994
(51) Int. Cl.: A61K 7/135, A45D 19/00

(54) **Procédé de décoloration des fibres kératiniques humaines à l'aide de vapeur d'eau**
Verfahren zum Blondieren von Menschlichen Keratinfasern mit Wasserdampf
Process for hair bleaching using steam

(30) Priorité: 22.12.1993 FR 9315484
(43) Date de publication de la demande: 28.06.1995
(73) Titulaire: L'OREAL, 75008 Paris (FR)
(72) Inventeur: Samain, Henri, F-91570 Bievres (FR); Sturla, Jean-Michel, F-92210 Saint-Cloud (FR)
(74) Mandataire: Andral, Christophe André Louis

(56) Documents cités:
- CH-A- 357 161
- DE-A- 1 025 590
- FR-A- 2 273 492
- US-A- 3 086 534

## Description

La présente invention est relative à un procédé de décoloration des fibres kératiniques mettant en oeuvre de la vapeur d'eau et une composition comprenant au moins un agent oxydant.

Il est bien connu de décolorer les fibres kératiniques, en particulier les cheveux humains, avec des compositions contenant un agent oxydant. En quelques minutes, voire en une heure, la couleur des cheveux tend vers le blond.

Cependant, on observe généralement que les cheveux ainsi traités présentent également un reflet roux. Ce reflet roux est souvent jugé comme inesthétique.

La demanderesse a donc recherché un moyen rapide de décolorer les cheveux naturels ou teints permettant de remédier à cet inconvénient.

La demanderesse a maintenant découvert de façon surprenante que l'utilisation d'un gaz chauffé à une température d'au moins 75°C comprenant de la vapeur d'eau sur des cheveux traités avec une composition comprenant au moins un agent oxydant permettait d'obtenir une décoloration rapide et n'engendrant aucun reflet roux.

Les cheveux présentent d'autre part d'excellentes qualités cosmétiques finales.

On notera que l'utilisation de la vapeur d'eau dans des procédés de coloration ou de décoloration d'oxydation a déjà été décrite dans le brevet FR1011151, document dans lequel de la vapeur d'eau chauffée à environ 50°C est mise en oeuvre dans le but d'accélérer le processus de coloration ou de décoloration des cheveux. Toutefois, à cette température, ce procédé ne permet pas de diminuer les reflets roux décrits ci-dessus.

Le brevet CH-A-357 161 décrit un procédé de décoloration des cheveux consistant à appliquer le produit traitant sur les cheveux, exposant les cheveux à de la vapeur d'eau enrichie en ozone, dans un casque englobant toute la partie chevelue de la tête. De même, le brevet US-A-3 086 534 décrit un procédé de décoloration des cheveux, caractérisé par le fait que la composition décolorante est appliqué sur les cheveux et ceux-ci sont traités par un mélange de vapeur d'eau et d'ozone en plaçant la tête chevelue sous un casque.

La présente invention concerne donc un procédé de décoloration des fibres kératiniques, caractérisé par le fait qu'il consiste à mettre en contact des fibres kératiniques sur lesquelles on a appliqué une composition décolorante contenant au moins un agent oxydant, avec un gaz contenant de la vapeur d'eau, la température dudit gaz étant supérieure à 75°C, le temps de contact entre ledit gaz et lesdites fibres à décolorer étant inférieur à dix minutes.

En plus de la vapeur d'eau, le gaz vecteur peut contenir de la vapeur de solvant, des gaz tels que l'oxygène, l'azote, des mélange de gaz tels que l'air ou d'autres composés vaporisables.

Les solvants utilisables pour la production de vapeur sont des solvants organiques cosmétiquement acceptables et plus particulièrement des alcools tels que l'éthanol, l'isopropanol, l'alcool benzylique, l'alcool phényléthylique ou des glycols ou éthers de glycol tels que par exemple les éthers monométhylique, monoéthylique et monobutylique de l'éthylène glycol, le propylène glycol, le butylène glycol, le dipropylèneglycol ainsi que les alkyléthers comme le monobutyléther du diéthylèneglycol.

Le gaz comprend de préférence au moins 1% en volume de vapeur d'eau par rapport au volume total du gaz.

Le gaz est constitué de préférence soit uniquement ou essentiellement de vapeur d'eau, soit de mélanges de vapeur d'eau et d'air.

La température du gaz est de préférence supérieure ou égale à 85°C et plus particulièrement comprise entre 85 et 150°C.

De préférence. le gaz est mis en contact avec la fibre pendant une durée allant de 0,01 seconde à 5 minutes et encore plus préférentiellement de 10 secondes à 3 minutes.
L'application du gaz peut être répétée plusieurs fois sur la même fibre. chaque opération se faisant selon la durée indiquée ci-dessus.

Dans un premier mode de mise en oeuvre du procédé selon l'invention qui est ici préféré, on applique sur les cheveux une composition de décoloration capillaire contenant un agent oxydant, puis on les soumet à l'action de la vapeur d'eau.

Selon d'autres modes de mise en oeuvre du procédé, il est également possible d'appliquer simultanément la composition décolorante et le gaz comprenant de la vapeur d'eau.

Il est également possible de faire parvenir sur les cheveux tout ou partie de la composition décolorante à l'aide du flux de gaz lorsque certains ou tous les constituants de la composition sont entraînables ou vaporisables.

Dans un mode particulier de réalisation de l'invention, l'application de vapeur d'eau est suivie d'un rinçage à l'eau.

La production d'un gaz chaud comprenant de la vapeur d'eau peut se faire à l'aide de tout appareil connu en soi. Toutefois, selon la présente invention, on utilise de préférence un appareil tel que celui décrit dans le brevet français FR-A-2273492, ou tout autre appareil équivalent, qui convient particulièrement bien.

L'agent oxydant est choisi de préférence parmi le peroxyde d'hydrogène, le peroxyde d'urée, les bromates de métaux alcalins, les persels tels que les perborates et les persulfates, les peracides organiques comme le monoperoxyphtalate de magnésium. Le peroxyde d'hydrogène est particulièrement préféré.

L'agent oxydant est présent dans des concentrations allant de 0,01 à 10% en poids par rapport au poids total de la composition.

La composition décolorante peut avoir un pH compris entre 3 et 11,5, qui peut être ajusté à la valeur choisie au moyen d'agents alcalinisants habituellement utilisés en décoloration des fibres kératiniques, tels que l'ammoniaque, les carbonates alcalins, les alcanolamines, par exemple les mono-, di - et triéthanolamines ainsi que leurs dérivés, les hydroxydes de sodium ou de potassium, ou d'agents acidifiants classiques, tels que les acides minéraux ou organiques comme par exemple les acides chlorhydrique, tartrique, citrique et phosphorique.

Les compositions de décoloration contiennent également, dans leur forme de réalisation préférée, des agents tensioactifs anioniques, cationiques, nonioniques, amphotères, bien connus de la technique, ou leurs mélanges, dans des proportions comprises entre environ 0,5 et 55 % en poids, et de préférence entre 2 et 50 % en poids par rapport au poids total de la composition.

Elles peuvent également contenir des solvants organiques pour solubiliser les composants qui ne seraient pas suffisamment solubles dans l'eau. Parmi eux, on peut citer à titre d'exemple, les alcanols inférieurs en C₁-C₄, tels que l'éthanol et l'isopropanol, le glycérol, les glycols ou éthers de glycols comme le 2-butoxyéthanol, le propylène glycol, le monoéthyléther et le monométhyléther du diéthylèneglycol, ainsi que les alcools aromatiques comme l'alcool benzylique ou le phénoxyéthanol, les produits analogues et leurs mélanges.

Ces solvants sont présents de préférence dans des proportions comprises entre environ 1 et 40 % en poids, et en particulier entre 5 et 30 % en poids par rapport au poids total de la composition.

On peut aussi ajouter des agents épaississants choisis par exemple parmi les polymères d'acide acrylique éventuellement réticulés ou des agents épaississants minéraux tels que la bentonite. présents de préférence dans des proportions comprises entre environ 0,1 et 5 %, et en particulier entre 0,2 et 3 % en poids par rapport au poids total de la composition.

Des agents antioxydants peuvent également être introduits. lls sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la 2-tertiobutyl hydroquinone et l'acide homogentisique et sont présents dans des proportions comprises entre environ 0,05 et 1,5 % en poids par rapport au poids total de la composition.

Les compositions de décoloration peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition de décoloration utilisée dans le procédé selon l'invention peut se présenter sous des formes habituellement utilisées pour la décoloration des cheveux telles que de liquide plus ou moins épaissi ou gélifié, de crème, de mousse en aérosol ou sous toute autre forme appropriée cour réaliser une décoloration des cheveux.

Les exemples qui suivent illustrent l'invention sans pour autant présenter un caractère limitatif.

### Exemple 1

Sur deux mèches de cheveux châtain fonce, on a applique un même mélange éclaircissant de composition suivante (ce mélange a été obtenu en mélangeant la composition A à la composition B, puis mélange à l'eau oxygénée) :

| | |
|---|---|
| - Composition A | 60 g |
| - Composition B | 50 g |
| - Eau oxygénée 30 volumes (pH 3) | 120 ml |

avec :

### Composition A :

| | |
|---|---|
| - Nonylphénol oxyéthyléné à 4 moles d'oxyde d'éthylène | 25 g |
| - Nonylphénol oxyéthyléné à 9 moles d'oxyde d'éthylène | 20 g |
| - Acide laurique oxyéthyléné à 2 moles d'oxyde d'éthylène | 7 g |
| - Acide oléique | 30g |
| - Propylène glycol | 12 g |
| - Ammoniaque (20% de NH₃) | 7 g |
| - Eau déminéralisée | 100 g |

### Composition B :

| | |
|---|---|
| - Persulfate de sodium | 30 g |
| - Persulfate de potassium | 25 g |
| - Disilicate de sodium anhydre | 3 g |
| - Silice colloïdale | 0,4 g |
| - Chlorure d'ammonium | 12 g |

L'une (mèche n°1) a subi un traitement classique : 30 mn à température ambiante. L'autre (mèche n°2) a subi un traitement à la vapeur d'eau selon l'invention, à savoir un traitement pendant 1 mn 30 s avec de la vapeur d'eau a 90°C.

Les résultats de décoloration, qui sont appréciés ici par la mesure des coordonnées chromatiques de chacune des deux mèches dans le système L.a.b (colorimètre MINOLTA CHROMA METER CR 200), sont les suivants :

| | L | a | b |
|---|---|---|---|
| mèche n°1 | 34,15 | 11 | 19,7 |
| mèche n°2 | 34,1 | 9,4 | 18,5 |

Les éclaircissements sont équivalents (L à peu prés égaux), mais les nuances sont moins rousses dans le cas des cheveux traités à la vapeur (les valeurs a et b sont plus faibles, la nuance est en conséquence moins rouge et moins jaune).

### Exemple 2

Sur deux mèches de cheveux châtain foncé, on a appliqué le même mélange eclaircissant que celui de l'exemple 1.

L'une (mèche n°1) a subi deux traitements : deux fois une heure à température ambiante.
L'autre (mèche n°2) a subi un traitement à la vapeur d'eau selon l'invention, à savoir deux traitements pendant 2 mn chacun avec de la vapeur d'eau à 90°C.

Les résultats de décoloration (colorimetre MINOLTA CHROMA METER CR 200) sont les suivants :

| | L | a | b |
|---|---|---|---|
| mèche n°1 | 59,7 | 7,3 | 31,9 |
| mèche n°2 | 59,8 | 5,8 | 27 |

Comme pour l'exemple 1, les éclaircissements sont équivalents (L à peu près égaux), mais les nuances sont moins rousses dans le cas des cheveux traités à la vapeur (mèche n°2).

## Revendications

1. Procédé de décoloration des fibres kératiniques, caractérisé par le fait qu'il consiste à mettre en contact des fibres kératiniques sur lesquelles on a appliqué une composition décolorante contenant au moins un agent oxydant, avec un gaz contenant de la vapeur d'eau, la température dudit gaz étant supérieure à 75°C, le temps de contact entre ledit gaz et lesdites fibres à décolorer étant inférieur à dix minutes.

2. Procédé selon la revendication 1, caractérisé par le fait que le gaz a une température supérieure ou égale à 85°C.

3. Procédé selon la revendication 2, caractérisé par le fait que le gaz a une température comprise entre 85 et 150°C.

4. Procédé selon l'une quelconque des revendications précédentes, caractérisé par le fait que le gaz est mis en contact avec la fibre à décolorer pendant une durée allant de 0,01 seconde à 5 minutes.

5. Procédé selon la revendication 4, caractérisé par le fait que le gaz est mis en contact avec la fibre à décolorer pendant une durée allant de 10 seconde à 3 minutes.

6. Procédé selon l'une quelconque des revendications précédentes, caractérisé par le fait que l'application du gaz est répétée plusieurs fois sur une même fibre.

7. Procédé selon l'une quelconque des revendications précédentes, caractérisé par le fait que le gaz contient uniquement de la vapeur d'eau.

8. Procédé selon l'une quelconque des revendications 1 à 6, caractérisé par le fait que le gaz contient de la vapeur d'eau et au moins un autre composé sous forme de gaz ou de vapeur.

9. Procédé selon la revendication 8, caractérisé par le fait que le gaz contient de la vapeur d'eau et de l'air.

10. Procédé selon l'une quelconque des revendications précédentes, caractérisé par le fait que l'agent oxydant est choisi parmi le peroxyde d'hydrogène, le peroxyde d'urée, les bromates de métaux alcalins, les persels tels que les perborates et les persulfates, les peracides organiques comme le monoperoxyphtalate de magnésium.

11. Procédé selon l'une quelconque des revendications précédentes, caractérisé par le fait que l'agent oxydant est présent dans des concentrations allant de 0,01 à 10% en poids par rapport au poids total de la composition.

## Claims

1. Process for bleaching keratinous fibres, characterized in that it consists in bringing keratinous fibres, to which a bleaching composition containing at least one oxidizing agent has been applied, into contact with a gas containing water vapour, the temperature of the said gas being greater than 75°C and the contact time between the said gas and the said fibres to be bleached being less than ten minutes.

2. Process according to Claim 1, characterized in that the gas has a temperature greater than or equal to 85°C.

3. Process according to Claim 2, characterized in that the gas has a temperature between 85 and 150°C.

4. Process according to any one of the preceding claims, characterized in that the gas is brought into contact with the fibre to be bleached for a period of time ranging from 0.01 second to 5 minutes.

5. Process according to Claim 4, characterized in that the gas is brought into contact with the fibre to be bleached for a period of time ranging from 10 seconds to 3 minutes.

6. Process according to any one of the preceding claims, characterized in that application of the gas to the same fibre is repeated a number of times.

7. Process according to any one of the preceding claims, characterized in that the gas contains solely water vapour.

8. Process according to any one of Claims 1 to 6, characterized in that the gas contains water vapour and at least one other compound in the form of gas or vapour.

9. Process according to Claim 8, characterized in that the gas contains water vapour and air.

10. Process according to any one of the preceding claims, characterized in that the oxidizing agent is chosen from hydrogen peroxide, urea hydrogen peroxide, alkali metal bromates, persalts such as perborates and persulphates, or organic peracids such as magnesium monoperoxyphthalate.

11. Process according to any one of the preceding claims, characterized in that the oxidizing agent is present in concentrations ranging from 0.01 to 10% by weight with respect to the total weight of the composition.

## Patentansprüche

1. Verfahren zum Blondieren von Keratinfasern, dadurch gekennzeichnet, daß das Verfahren darin besteht, daß man Keratinfasern, auf die eine Blondierzusammensetzung mit einem Gehalt an mindestens einem Oxidationsmittel aufgebracht ist, mit einem Wasserdampf enthaltenden Gas, dessen Temperatur über 75°C liegt, in Kontakt bringt, wobei die Kontaktzeit zwischen dem Gas und den zu blondierenden Fasern unter 10 Minuten liegt.

2. Verfahren nach Anspruch 1, dadurch gekennzeichnet, daß das Gas eine Temperatur von 85°C oder darüber hat.

3. Verfahren nach Anspruch 2, dadurch gekennzeichnet, daß das Gas eine Temperatur von 85 bis 150°C aufweist.

4. Verfahren nach einem der vorstehenden Ansprüche, dadurch gekennzeichnet, daß das Gas mit einer zu blondierenden Faser für eine Zeitspanne von 0,01 Sekunden bis 5 Minuten in Kontakt gebracht wird.

5. Verfahren nach Anspruch 4, dadurch gekennzeichnet, daß das Gas für eine Zeitspanne von 10 Sekunden bis 3 Minuten mit der zu entfärbenden Faser in Kontakt gebracht wird.

6. Verfahren nach einem der vorstehenden Ansprüche, dadurch gekennzeichnet, daß die Gaseinwirkung mehrfach auf der gleichen Faser wiederholt wird.

7. Verfahren nach einem der vorstehenden Ansprüche, dadurch gekennzeichnet, daß das Gas nur Wasserdampf enthält.

8. Verfahren nach einem der Ansprüche 1 bis 6, dadurch gekennzeichnet, daß das Gas Wasserdampf und mindestens eine weitere Verbindung in Form von Gas oder Dampf enthält.

9. Verfahren nach Anspruch 8, dadurch gekennzeichnet, daß das Gas Wasserdampf und Luft enthält.

10. Verfahren nach einem der vorstehenden Ansprüche, dadurch gekennzeichnet, daß das Oxidationsmittel unter Wasserstoffperoxid, Harnstoff-Wasserstoffperoxid, Alkalimetallbromaten, Persalzen, wie Perboraten und Persulfaten, und organischen Persäuren, wie Magnesiummonoperoxyphthalat, ausgewählt wird.

11. Verfahren nach einem der vorstehenden Ansprüche, dadurch gekennzeichnet, daß das Oxidationsmittel in Konzentrationen von 0,01 bis 10 Gew.-%, bezogen auf das Gesamtgewicht der Zusammensetzung, enthalten ist.
